# EUROPÄISCHE PATENTANMELDUNG

(11) **EP 0 583 592 A2**
(43) Veröffentlichungstag der Anmeldung: **23.02.1994**
(21) Anmeldenummer: 93110710.6
(22) Anmeldetag: 05.07.1993
(51) Int. Cl.: G01N 33/38

(54) **Vorrichtung zum Erfassen der verarbeitungsoffenen Zeit und der Zeit und des Ausmasses des Stellens und Ribbelns von Putzmörtel**

(30) Priorität: 28.07.1992 DE 4224905
(71) Anmelder: HOECHST AKTIENGESELLSCHAFT, D-65926 Frankfurt am Main (DE)
(72) Erfinder: Schermann, Walter, Dr., D-6500 Mainz (DE); Podest, Helmut, A-5431 Kuchl (AT)

(57) **Zusammenfassung**

Bei der Vorrichtung zum Erfassen der verarbeitungsoffenen Zeit und der Zeit und des Ausmaßes des Stellens und Ribbelns von Putzmörtel sind auf einem Maschinenbett (1) ein Träger (10) für den Putzmörtel (3), der von einer verstellbaren Dreipunktauflage (8a, 8b, 8c) unterstützt wird und ein längs zum Träger (10) verschiebbarer Werkzeughalter (2), der eine zweidimensional verschiebbare Spanneinrichtung (5) aufweist, angeordnet. Der Werkzeughalter (2) wird durch eine Führung (9) geführt, die im Maschinenbett (1) angeordnet ist und die Spanneinrichtung (5) ist mit Halterungen (18, 19, 21) für Prüfwerkzeuge (20, 25), für eine optische Erkennungseinrichtung (22, 23) und für eine Tasteinrichtung (17) versehen.

## Beschreibung

Die Erfindung betrifft eine Vorrichtung nach dem Oberbegriff des Patentanspruchs.

Putzmörtel, insbesondere Maschinenputzmörtel zeigen in jeder Applikationsphase differenzierte Verhaltensweisen. Darum ist es wichtig, das Eigenschaftsspektrum beim Spritzen, Aufziehen, Verteilen, Abziehen, Filzen und Glätten möglichst gut zu kennen. Das Eigenschaftsspektrum wird allerdings nicht nur durch die Mörtelrezeptur, sondern auch von den Wechselwirkungen der frisch applizierten Mörtelmasse mit dem saugfähigen Untergrund geprägt. Die Verarbeitungskonsistenz eines Putzmörtels wird im Labor anhand des sogenannten Ausbreitmaßes bestimmt. Dynamische Konsistenzkontrollen mit Drehmomentrührwerken sind ebenfalls bekannt.

Der auf die Wand aufgetragene Putzmörtel wird innerhalb der verarbeitungsoffenen Zeit weiter bearbeitet, nämlich verteilt und abgezogen. In diesem Zeitraum, der durch das sog. Stellen nach oben hin begrenzt wird, nimmt die Mörtelkonsistenz stetig zu. Das Zeitintervall zwischen Putzauftrag und Stellen wird per Hand mit einem Spatel ermittelt. Hierzu wird der Spatel leicht in die Putzmörteloberfläche gedrückt und parallel zur Oberfläche schabend geführt. Anhand mehrerer Versuche kann der Zeitpunkt des Stellens eingegrenzt und das entstandene Rauhigkeitsmuster qualitativ beschrieben werden. Nach der Stellzeit, d.h. nach Verfestigung des Putzgefüges, wird der aufgezogene Putzmörtel geschnitten, gefilzt und geglättet. Er darf dabei nicht aufreißen. Das Stellen geht mit zunehmender Verfestigung des Putzmörtels in Ribbeln über. Nachteilig ist, daß dokumentierbare, objektive Meßdaten für diese für das Verarbeiten des Putzmörtels wichtigen Zeitintervalle nicht erhalten werden.

Hier will die Erfindung Abhilfe schaffen. Es soll eine Vorrichtung zur Verfügung gestellt werden, mit der die Herstellung und Verarbeitung von Putzmörtel überwacht werden kann und die Meßdaten liefert, die mit dem Verhalten des Putzmörtels auf der Baustelle korrelieren und Vergleiche zu früheren Meßreihen zulassen.

Die Aufgabe wird durch eine Vorrichtung der eingangs genannten Art gelöst, die dadurch gekennzeichnet ist, daß auf einem Maschinenbett ein Träger für den Putzmörtel, der von einer verstellbaren Dreipunktauflage unterstützt wird und ein längs zum Träger verschiebbarer Werkzeughalter, der eine zweidimensionale verschiebbare Verspanneinrichtung aufweist, angeordnet sind, wobei der Werkzeughalter durch eine Führung geführt wird, die im Maschinenbett angeordnet ist und die Spanneinrichtung mit Halterungen für Prüfwerkzeuge, für eine optische Erkennungseinrichtung und für eine Tasteinrichtung versehen ist.

Bei einer alternativen Ausgestaltung wird statt des Werkzeughalters der Träger mit der Dreipunktauflage auf einem Schlitten angeordnet, der durch eine Führung im Maschinenbett geführt wird.

Die Prüfwerkzeuge können aus einem Prüfrad und mindestens einem Prüfspatel bestehen. Die optische Erkennungseinrichtung kann aus zwei optischen Sensoren üblicher Art bestehen, die in Kreuzmodulationsschaltung miteinander verknüpft sind.

Die Vorteile der erfindungsgemäßen Vorrichtung sind im wesentlichen darin zu sehen, daß objektive Meßgrößen und Beurteilungskriterien für die einzelnen kontinuierlich aufeinander folgenden Applikationsphasen eines Putzmörtels bereitgestellt werden. Insbesondere ergeben sich quantitative Informationen über die verarbeitungsoffene Zeit (Zeitraum zwischen Putzapplikation und Beginn des Stellens), Konsistenzveränderungen, temporärer Zusammenhalt des Partikelhaufwerkes - insbesondere Ausmaß des Stellens und Auswirkungen mechanischer Nachbearbeitungen in der Schneidphase (Ribbeln). Damit wird der Putzmörtelhersteller in die Lage versetzt, Anwendungseigenschaften zu optimieren und dem Handwerker die Verarbeitung zu erleichtern.

Im folgenden wird die Erfindung anhand von lediglich einen Ausführungsweg darstellende Figuren näher erläutert.

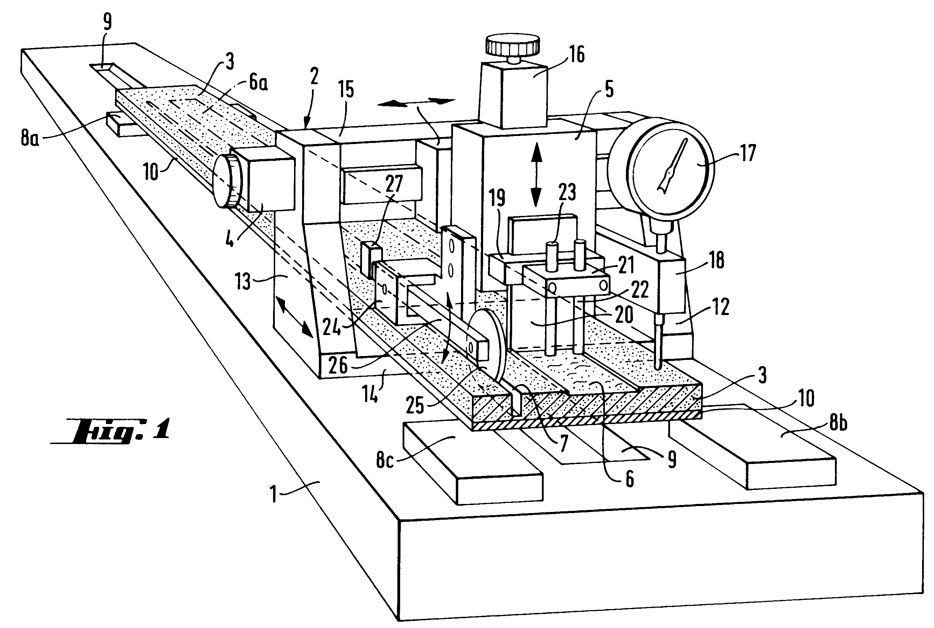

Es zeigt
- Figur 1: die Vorrichtung perspektivisch ohne Spanneinrichtung;
- Figur 2: eine Alternative zur Vorrichtung gemäß Figur 1;

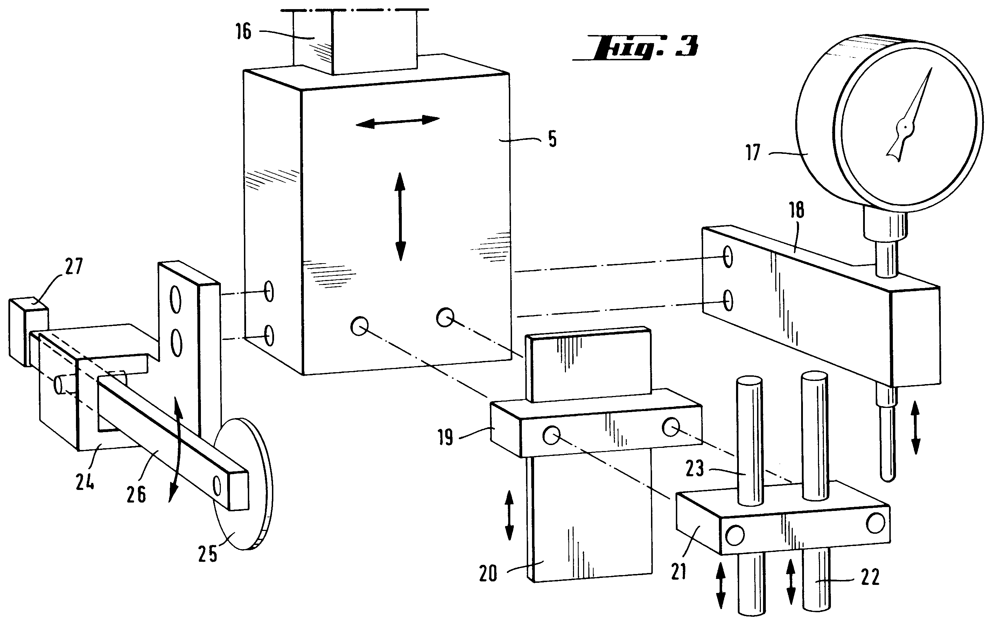

- Figur 3: die Spanneinrichtung mit Prüfwerkzeuge explodiert dargestellt und
- Figur 4a,b: den Prüfspatel in zwei Ausführungsformen.

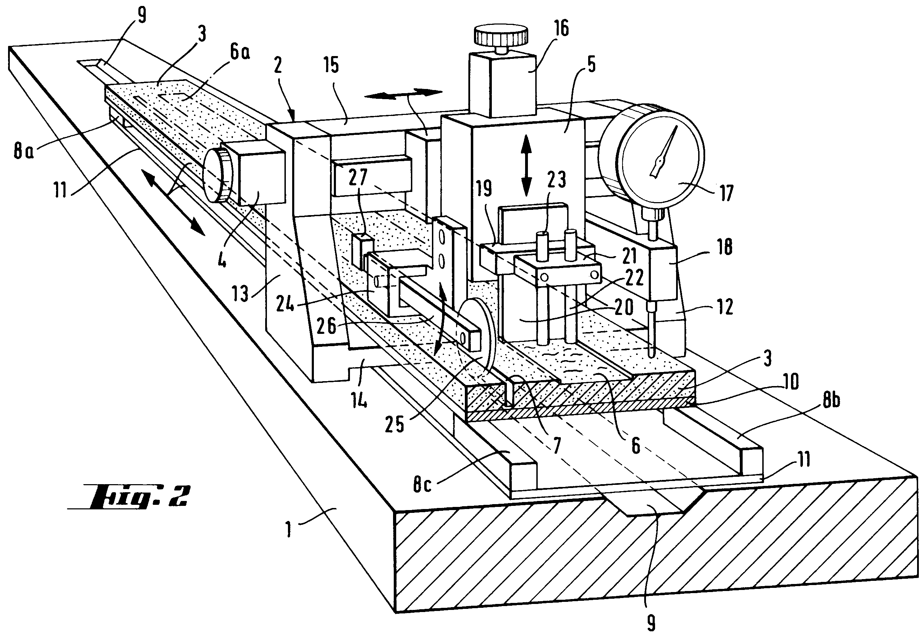

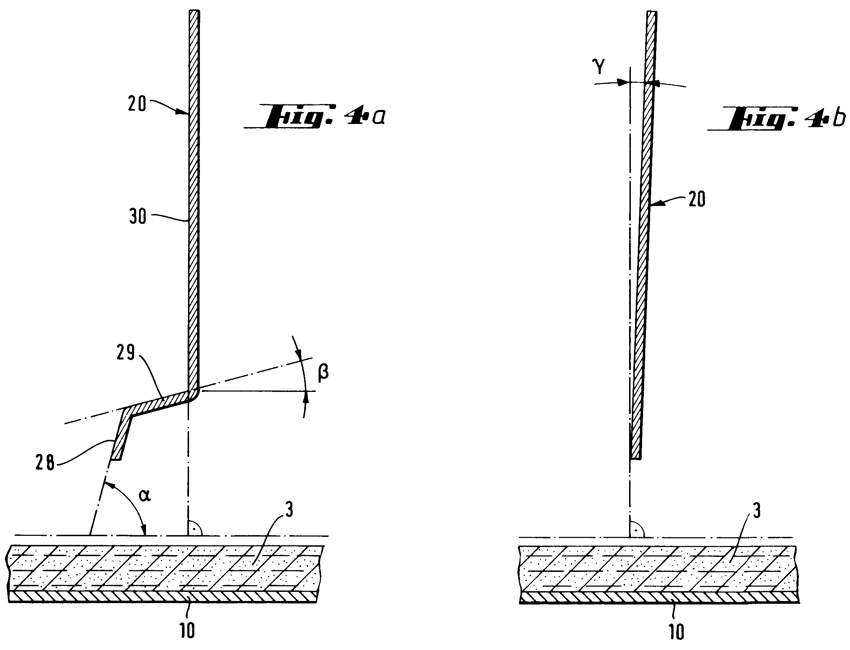

Auf einem Maschinenbett 1 sind ein Träger 10 für den Putzmörtel 3 und ein Werkzeughalter 2 angeordnet. Der Träger 10 kann aus einem präparierten Gasbeton-, Ziegelbaustein oder dergleichen bestehen. Der Träger 10 wird durch eine Dreipunktauflage 8a, 8b, 8c unterstützt. Damit kann die Mörtelschicht in eine zum Maschinenbett 1 planparallele Lage gebracht werden. Der Werkzeughalter 2 ist auf dem Maschinenbett 1 verschiebbar angeordnet, wie mit Pfeil angedeutet. Er wird dabei durch die Führung 9 im Maschinenbett 1 geführt. Der Werkzeughalter 2 kann auch stationär auf dem Maschinenbett 1 angeordnet sein. In diesem Fall ist der Träger 10 mit der Dreipunktauflage 8a, 8b, 8c auf einem Schlitten 11 (Figur 2) montiert, der dann durch Führung 9 geführt wird. Werkzeughalter 2 und Schlitten 11 können manuell oder motorisch (nicht dargestellt) verschoben werden. Der Werkzeughalter 2 besteht aus den Böcken 12, 13 und den Traversen 14, 15, wobei die Traverse 14 einen in die Führung ragende Nocken oder dergleichen (nicht dargestellt) aufweisen kann und die Traverse 15 mit einer transversal verlaufenden Verschiebeeinrichtung 4 versehen ist. Die Verschiebeeinrichtung 4 trägt die Spanneinrichtung 5 mit vertikaler Verschiebeeinrichtung 16. Sowohl die transversale 4 als auch die vertikale Verschiebeeinrichtung 16 können manuell oder motorisch bewegt werden. Die Spanneinrichtung 5 mit vertikaler Verschiebeeinrichtung 16 trägt die Prüfwerkzeuge. Tasteinrichtung 17 ist in Halterung 18 verschiebbar angeordnet. Halterung 19 trägt den Prüfspatel 20 zum Stellen/Ribbeln des Mörtels. Halterung 19 trägt auch Halterung 21 für die optische Erkennungseinrichtung bestehend aus optischen Sensoren 22, 23. Die Grobeinstellung der Sensoren 22, 23 und des Prüfspatels 20 erfolgt in ihrer jeweiligen Halterung, während die Feineinstellung über die Verschiebeeinrichtungen 4 und 16 vorgenommen wird. Figur 4 zeigt mögliche Formen für den Prüfspatel 20, wobei die Version (a) sich besonders gut zum Stellen eignet, während die Version (b) Vorzüge beim Ribbeln aufweist. Spatel (a) kann folgende Konstruktionsmerkmale besitzen: Breite 20 mm, Länge der Schneid-/Stellfläche 28 ca. 3 mm, Länge der Rutschfläche 29 ca. 1 - 5 mm, Anstellwinkel α der Stellfläche zwischen 80° und 85°, Neigung β der Rutschfläche 5° bis 15°, Länge der Einspannfläche 30 ca. 20 - 80 mm (abhängig von der Elastizität des Spatelmaterials). Spatel (b) kann gegen die Vertikale um bis zu ± 2° geneigt sein (Winkel γ). Die beiden Spatelformen können auch nebeneinander auf der Halterung 19 angeordnet sein. Mit zunehmender Verfestigung des Mörtels geht das Stellen allmählich in Ribbeln über, d.h. die Stellspur 6 wird immer flacher und geht allmählich in eine Ribbelspur 6a über. Analog entwickelt sich der Prüfradeindruck 7. Ist der Mörtel 3 fest, gleitet das Prüfrad 25 auf der Mörteloberfläche. Das Prüfrad ist an einem Ende eines Hebels 26 befestigt. Am anderen Ende trägt der Hebel 26 ein Gegengewicht 27. Der Hebel 26 selbst ist in Halterung 24 drehbar gelagert.

Nach Auftragen des Putzmörtels auf den Träger 10 und nivellieren der Putzmörteloberfläche werden das Prüfrad 25, der Prüfspatel 20 sowie die optischen Sensoren in Stellung gebracht, d.h. das Prüfrad 25 wird z.B. bis auf den Träger 10 und der Prüfspatel 20 z.B. 3 mm in die Putzmörtelschicht abgesenkt, die optischen Sensoren (z.B. Glasfaserreflextaster vom Typ FSA 30 U - 50 der Firma Schlüter Fotooptik GmbH + Co KG, D-7861 Wieden) bis auf ca. 10 mm über der Mörteloberfläche. Die optischen Sensoren 22, 23 sind vorzugsweise in Kreuzmodulation geschaltet. Das Einstellen der Prüfwerkzeuge und der optischen Erkennungseinrichtung geschieht mit Hilfe der Tasteinrichtung 17 und den Verschiebeeinrichtungen 4 und 16. Nach dem Einstellen der Prüfwerkzeuge und der optischen Erkennungseinrichtung wird der aufgetragene Putzmörtel unter dem Werkzeughalter mit einer Geschwindigkeit, die im wesentlichen von der Mörtelzusammensetzung abhängt, von 10 cm bis 40 cm/h durchgezogen. Das Prüfrad 25 hinterläßt dabei den Prüfradeindruck 7, der mit zunehmender Mörtelkonsistenz auf Null abnimmt (Ende der verarbeitungsoffenen Zeit). Simultan schiebt der Prüfspatel 20 während dieser Zeit Mörtelbrei vor sich her, der sich mit zunehmender Konsistenz immer mehr stellt, bis er spanend abgetragen wird. Zu diesem Zeitpunkt beginnt die Stellzeit, die dann beendet ist, wenn kein Span mehr erzeugt wird, d.h. der Prüfspatel 20 über die Oberfläche kratzt (ribbelt). Während der offenen Zeit wird der Putzmörtel aufgetragen, verteilt und gerichtet, während der Stellzeit geschnitten, anschließend gefilzt und geglättet.

Mit der optischen Erkennungseinrichtung werden die durch die mechanische Bearbeitung resultierenden Oberflächenstrukturen defektiert und ausgewertet.

## Patentansprüche

1. Vorrichtung zum Erfassen der verarbeitungsoffenen Zeit und der Zeit und des Ausmaßes des Stellens und Ribbelns von Putzmörtel, dadurch gekennzeichnet, daß auf einem Maschinenbett (1) ein Träger (10) für den Putzmörtel (3), der von einer verstellbaren Dreipunktauflage (8a, 8b, 8c) unterstützt wird und ein längs zum Träger (10) verschiebbarer Werkzeughalter (2), der eine zweidimensional verschiebbare Spanneinrichtung (5) aufweist, angeordnet sind, wobei der Werkzeughalter (2) durch eine Führung (9) geführt wird, die im Maschinenbett (1) angeordnet ist und die Spanneinrichtung (5) mit Halterungen (18, 19, 21) für Prüfwerkzeuge (20, 25), für eine optische Erkennungseinrichtung (22, 23) und für eine Tasteinrichtung (17) versehen ist.

2. Vorrichtung zum Erfassen der verarbeitungsoffenen Zeit und der Zeit und des Ausmaßes des Stellens und Ribbelns von Putzmörtel, dadurch gekennzeichnet, daß auf einem Maschinenbett (1) ein bewegbarer Träger (10) für den Putzmörtel (3), der von einer verstellbaren Dreipunktauflage (8a, 8b, 8c) unterstützt wird und ein Werkzeughalter (2) der eine zweidimensional verschiebbare Spanneinrichtung (5) aufweist, angeordnet sind, wobei der Träger (10) mit der Dreipunktauflage (8a, 8b, 8c) auf einem Schlitten (11) angeordnet ist, der durch eine Führung (9) im Maschinenbett (1) geführt wird und die Spanneinrichtung (5) mit Halterungen (18, 19, 21) für Prüfwerkzeuge (20, 25), für eine optische Erkennungseinrichtung (22, 23) und eine Tasteinrichtung (17) versehen ist.

3. Vorrichtung nach Anspruch 1 oder 2, dadurch gekennzeichnet, daß die Prüfwerkzeuge (20, 25) aus einem Prüfrad (25), und einem Prüfspatel (20) bestehen.

4. Vorrichtung nach Anspruch 1 oder 2, dadurch gekennzeichnet, daß die optische Erkennungseinrichtung aus zwei optischen Sensoren (22, 23) bestehen, die in Kreuzmodulationsschaltung miteinander verknüpft sind.
